# EUROPEAN PATENT SPECIFICATION

(11) **EP 2 939 617 B1**
(45) Date of publication and mention of the grant of the patent: **09.11.2016**
(21) Application number: 15150508.8
(22) Date of filing: 08.01.2015
(51) Int. Cl.: A61B 17/32

(54) **Follicle punch for use with curled follicles**
Follikel-Punsch für den Einsatz mit krause Follikel
Follicule poinçon pour une utilisation avec follicules bouclées

(30) Priority: 28.04.2014 US 201461985347 P
(43) Date of publication of application: 04.11.2015
(73) Proprietor: Umar, Sanusi, Redondo Beach, CA 90277 (US)
(72) Inventor: Umar, Sanusi, Redondo Beach, CA 90277 (US)
(74) Representative: Dickerson, David

(56) References cited:
- WO-A1-2009/146068
- WO-A2-2007/100581
- NL-C1- 1 032 931

## Description

### Field of the Invention

This invention relates to surgical instruments and, more particularly, to a punch for extracting hair follicles from the skin.

### Background of the Invention

Hair transplantation is a surgical technique that involves moving skin containing hair follicles from one part of the body (the donor site) to bald or balding parts (the recipient site).

Hair naturally grows in follicles that contain groupings of 1 to 4 hairs, and transplant techniques typically move the 1-4 hair "follicular units" from the donor site to the recipient site. Hair follicles grow at an angle to the skin, pointing from anterior to posterior.

The follicles of hair are typically removed from the donor site using punches of between 0.7mm and 1.25mm in diameter. The punches are tubular bodies having a skin-contacting cutting edge, and are typically mounted in a tool that causes the punch to rotate as the punch is brought into contact with the donor site. Hair follicles are very easily damage during the removal process, and damaged follicles are unlikely to be successfully transplanted.

Curled follicles are extremely susceptible to damage by follicle punches and are therefore particularly difficult to extract for successful transplantation. Such follicles are curled beneath the skin and are easily cut and/or damaged by the advancing cutting edge of conventional punches as the punch penetrates the donor site's tissue.

Further information pertaining to the prior art can be found in WO 2009/146068 which forms the basis of the preamble of claims 1 and 15.

### Summary of the Invention Claims 1 and 15 define the scope of the invention and the dependant claims disclose the preferred embodiments.

The present invention provides a follicle punch in accordance with independent claims 1 and 15. Preferred embodiments of the invention are reflected in the dependent claims.

The claimed invention can be better understood in view of the embodiments described and illustrated in the present disclosure, *viz.* in the present specification and drawings. In general, the present disclosure reflects preferred embodiments of the invention.

The present disclosure proposes a punch that is particularly useful for removing curled hair follicles from a donor site comprises a generally tubular body disposed about a generally longitudinal axis and having a distal cutting end region terminating distally in a plurality of distally-extending circumferentially disposed, generally prong-like members carrying distally diverging cutting edges and separated by follicle-accommodating slits or slots. As used
herein, the terms "slit" and "slot" are both used because the primary difference, as used herein, is subjective when working with dimensions of the small magnitudes discussed herein.

According to the present disclosure, there is provided a follicle punch comprising: a generally tubular body disposed about a generally longitudinal axis between distal and proximal ends, and having a distal cutting end region terminating distally in an opposing pair of distally-extending, generally prong-like members having an anterior notch and a posterior notch therebetween, wherein the prong-like members each have a curved anterior cutting edge and a curved posterior edge meeting at a cutting tip.

Optionally, the anterior cutting edge of each prong-like member is convex, and the posterior cutting edge of each prong-like member is concave.

Advantageously, the cutting tips are generally aligned with each other in the longitudinal direction.

Conveniently, the cutting tips are aligned with each other in a direction transverse to the longitudinal axis.

Advantageously, one of said anterior and posterior notches extends proximately further than the other of said notches.

Conveniently, the posterior notch extends proximately further than the anterior notch.

Optionally, the posterior notch extends further than the anterior notch by a length in the range of 1 mm to 2 mm, inclusive.

Advantageously, posterior notch has a generally "V"-shaped distal segment, and a generally "U"-shaped proximal segment extending.proximally from the distal segment.

Conveniently, the U-shaped notch includes two proximally extending legs connected by a generally laterally extending base at the proximal end of the notch, and wherein at least a portion of the legs carry respective cutting edges formed by respective bevelled surfaces.

Optionally, the base of the U-shaped notch lacks a cutting edge formed by a bevelled cutting surface.

Advantageously, at least one of the notches is generally V-shaped.

Conveniently, said V-shaped notch includes two proximally extending legs that carry respective cutting edges formed by respective bevelled surfaces.

Optionally, the prong-like members include bevelled surfaces terminating at the cutting edges.

Advantageously, the bevelled surfaces are formed on the inside surface of the tubular punch body.

Conveniently, the cutting edge formed by the bevelled cutting surface is at the punch's outer diameter.

In practice, the punch is oriented during the extraction process at the donor site so that the curled hair root passes into, and is spared from the advancing cutting edge by, a slit as the punch is inserted into and penetrates the tissue. The punch may then be rotated slightly so that the cutting edges cut most of the tissue surrounding the follicle without making damaging contact with the follicle. It may be noted that a rotary motion may not be necessary and, if rotation is desired, it may be in one direction or be in the form of an oscillatory rotary movement, depending on characteristics of the donor site and targeted follicle.

The foregoing insertion process may be performed manually or under machine or computer control, and with or without the aid of an ultrasonic transducer coupled to the punch to impart a vibratory cutting force against the tissue. In addition, a mechanism for automatically rotating the punch may be employed, and may accordingly be coupled to the ultrasonic transducer if one is used.

So that the invention may be more readily understood, and so that further features thereof may be appreciated, embodiments will now be described by way of example with reference to the accompanying drawings in which:
Figure 1 is a front elevation view of a punch (known in the prior art) used for removing hair follicles.
Figure 2 is a side elevation view of a punch of Figure 1;
Figure 3 is a longitudinal section view of the punch of Figure 1, taken along line 3-3 in Figure 1;
Figure 4 is a longitudinal section view of the punch of Figure 1, taken along line 4-4 in Figure 2;
Figure 5 is an enlarged fragmentary view of the portion of the punch illustrated within the line 5 of Figure 4;
Figure 6 is a bottom plan view of the punch of Figure 2;
Figure 7 is a fragmentary view in perspective of the cutting end region of the punch oriented per line 7-7 in Figure 6 illustrated in Figure 1;
Figure 8 is an oblique fragmentary elevation view of the cutting end region of the punch of Figure 1;
Figure 9 is an oblique bottom view of the cutting end region of the punch of Figure 1;
Figure 10 is a schematic illustration of the prior art methodology for extracting a curled follicle;
Figure 11A is a right front oblique view, in schematic, of an embodiment of a punch that is constructed in accordance with the present disclosure for removing hair follicles;
Figure 11B is a longitudinal sectional view of the punch of Figure 11A, taken along line 11B-11B in Figure 11C;
Figure 11C is a front elevation view, in schematic, of the punch of Figure 11A,
Figure 12A is a right front oblique view, in schematic, of a punch that is constructed for removing hair follicles;
Figure 12B is a longitudinal sectional view of the punch of Figure 12A, taken along line 12B-12B in Figure 12C;
Figure 12C is a front elevation view, in schematic, of the punch of Figure 12A;
Figure 13A is a rear elevation view, in schematic, of the currently preferred embodiment of the distal end region of a punch constructed in accordance with the present disclosure for removing hair follicles from patients with curly or wavy hair;
Figure 13B is a left side elevation view, in schematic, of the distal end region illustrated in Figure 13A;
Figure 13C is a front elevation view, in schematic, of the distal end region illustrated in Figure 13A;
Figure 14A is a rear elevation view, in schematic, of the currently preferred embodiment of the distal end region of a punch constructed in accordance with the present disclosure for removing hair follicles from patients with kinky Afro-textured hair;
Figure 14B is a left side elevation view, in schematic, of the distal end region illustrated in Figure 14A;
Figure 14C is a front elevation view, in schematic, of the distal end region illustrated in Figure 14A; and
Figure 15 is a schematic illustration of the preferred methodology for extracting a curled follicle using the punch of Figures 14a-c in accordance with the present disclosure.

### Detailed Description

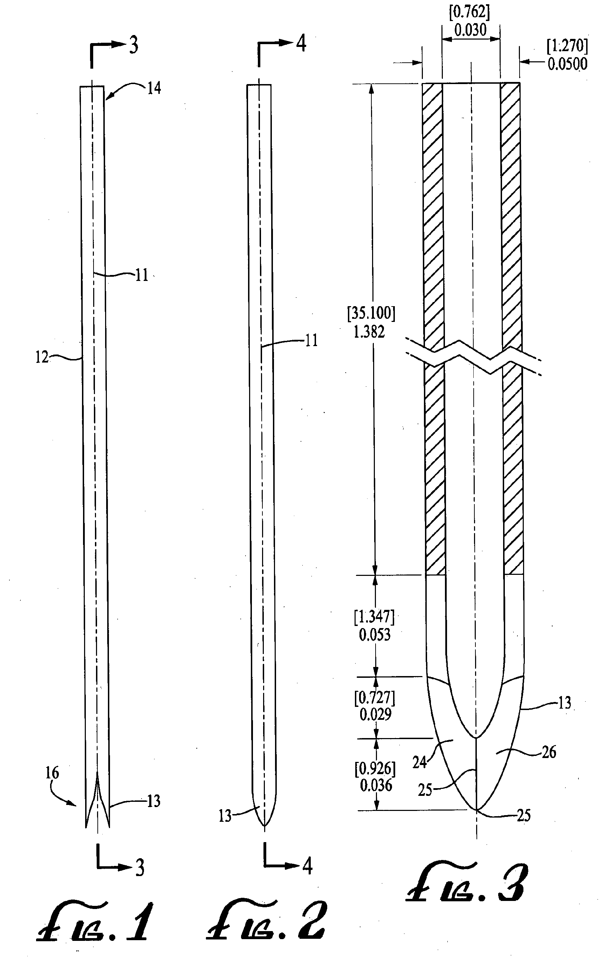

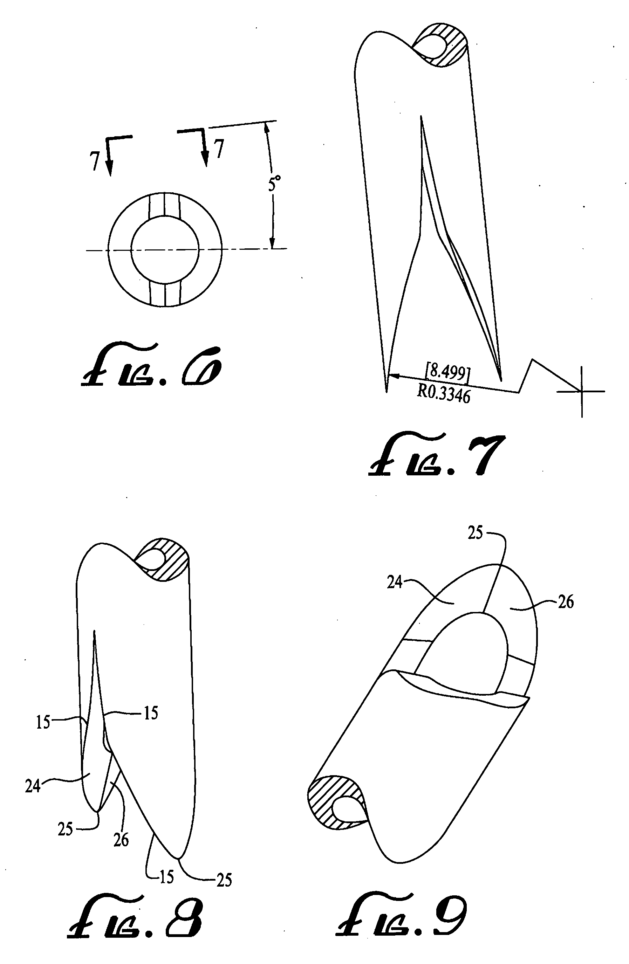

Referring to Figures 1-9, a punch for extracting curled follicles is illustrated as comprising a generally tubular body 12 extending from a proximal end 14 to a skin-contacting distal end
16 about a generally central longitudinal axis 11. The currently preferred dimensions of the punch are displayed in the Figures in both inches (unbracketed) and millimeters (bracketed), said currently preferred dimensions being part of this Detailed Description.

The punch's generally tubular body 12 has a distal cutting end region terminating distally in a plurality of distally-extending circumferentially disposed, generally prong-like members 13 carrying distally diverging cutting edges 15 separated by follicle-accommodating slits 22.

The currently preferred number of members 13 is two because a pair of such members currently appears to provide the appropriate amount of cutting around the follicle together with sufficient slit width to safely accommodate entry and protection of the follicle during the extraction process.

As illustrated in the Figures, each of the preferred prong-like members 13 has a generally convex outer surface and a generally concave inner surface substantially the same as that of the tubular body. As also illustrated in the Figures, each of the prong-like members also has a beveled cutting surface 24, 26 terminating at a cutting edge, with the bevel preferably being on the inside of the punch so that it terminates at a cutting edge on the punch's outer diameter. However, the formation of bevels on the outer surface of the punch is also possible, although not preferred, and is within the scope of the present disclosure. It may be noted that it is currently believed that the cutting edge portion illustrated in Figure 3 as totaling 1.653 mm in length may be as long as approximately 4 mm or so, and that the follicle- accommodating slit 22 should preferably be approximately 2 mm - 4 mm longer than the cutting edge portion.

The bevels 24, 26 are preferably created by grinding cutting edges outwardly from the interiors of the members 13 to produce sharp cutting edges. However, the bevels can also be formed by laser cutting, water jet or abrasive jet cutting, chemical moulding, and/or other manufacturing processes without departing from the scope of the present disclosure.

The bevels 24, 26 preferably interface at an apex of the prong-like member to provide a sharp, point-like, leading tip 25 which makes the initial penetration into the tissue that surrounds the targeted follicle, while the widening, generally semi-elliptical profiles of the prong-like members 13 cut more of the surrounding tissue as the punch is urged distally into the site. The leading tip can alternatively be a sharp rounded tip without departing from the scope of the present disclosure.

The distal end region of the punch may be further provided with generally circumferentially- extending notch having a generally concave shape that generally circumscribes the punch's outer surface. The notch preferably extends 1-2 mm proximally from a location closely adjacent the tip of the punch. The generally concave shape serves two purposes. First, its preferred size and shape results in a wound with everted edges; as the punch enters the tissue surrounding the targeted follicle, the tissue outward of the cut expands against the concavity as it is passed by the cutting edge. When the punch is subsequently withdrawn, the tissue resumes its consequently everted shape. Second, the generally concave shape and preferred sharpening from the inside of the punch results in a cutting force that is outwardly directed away from the follicle and tissue to be extracted, decreasing the risk of damage to the follicle.

Alternatively, the punch can be provided with a flared distal end having a diameter that has a diverging inner diameter and diverging outer diameter along the last 1 mm or so, with the flared end region resulting in a preferred gap of approximately 1.25 mm between opposing tips. Gaps of great or lesser spacing may be utilized as well, depending on the subject's hair and follicle dimensions without departing from the scope of the present disclosure.

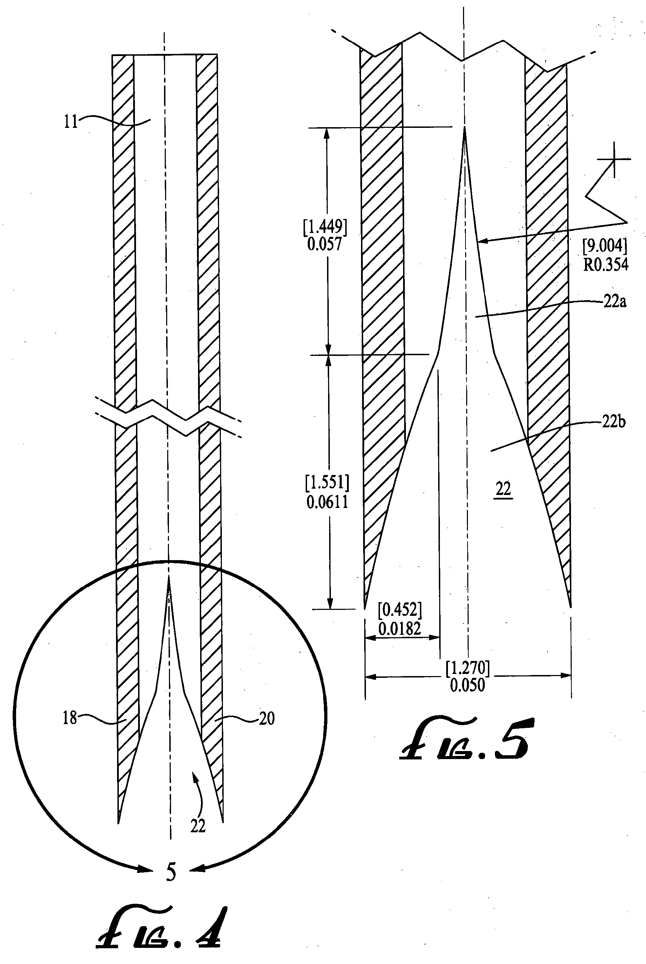

In one preferred configuration, the shape and dimensions of the slit 22 are, as best illustrated in Figures 4 and 5, a general inverted "V" profile having a relatively distal segment 22b and a relatively proximal segment 22a that is more steeply tapered than the distal segment 22b. The more steeply tapered interior of the relatively proximal segment provides a slit length and width that accommodates the follicle as the punch penetrates the surrounding tissue, in order to spare the follicle from being cut; the less tapered distal segment of the slit results in adequate spacing of the cutting edges of adjacent prong-like members 13 from the follicle's root structure so that the cutting yields a viable implant. Although the same taper could be used for both segments, it is preferable not to do so since a generally uniformly steep taper (such as that of the preferred distal segment) would add unnecessary length to the punch to achieve the needed spacing between the prongs, while a generally uniformly shallow taper (such as that of the preferred distal segment) would fail to provide the slit length needed.

The cutting edge of the punch, which preferably extends from its leading tip to the beginning of the steeply tapered portion of the slit (i.e., the interface of the proximal and distal slit segments), may be smooth or include one or more serrations. If serrations are included, it is
currently preferable that there be one or two serrations, with rounded edges, although the use of sharply angled edges would not depart from the scope of the present disclosure.

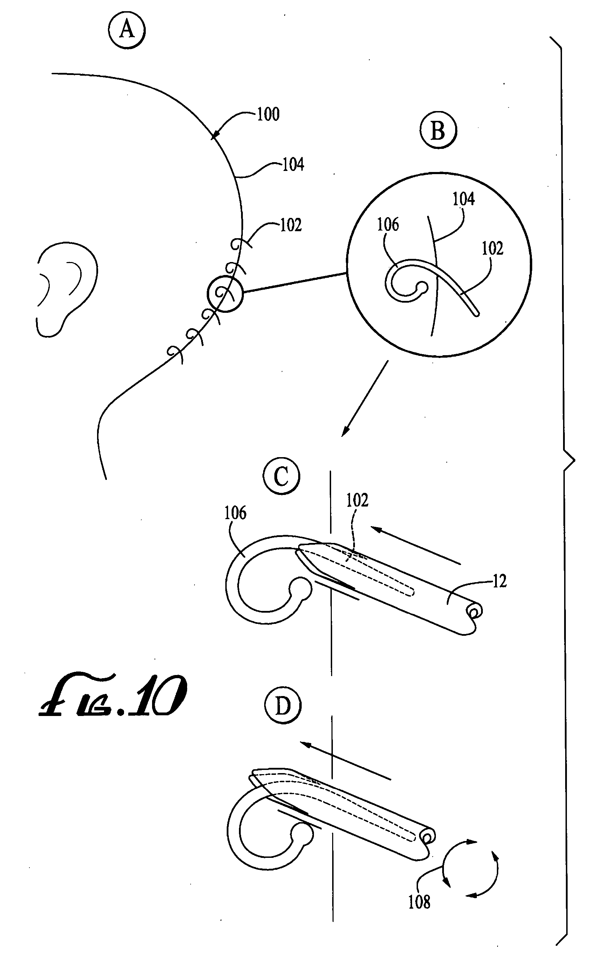

Figure 10 schematically illustrates (at "A") a subject's head 100 having a plurality of hairs 102 protruding from the skin 104. A hair 102 and its curled, subcutaneously-located follicle 106 is schematically illustrated in magnified form at "B".

As next schematically illustrated with greater magnification at "C", the punch is inserted into the skin at the donor site in such a way that the hair enters the punch's interior while the follicle 106 passes uncut through the slit 22. As further illustrated at "D", the punch is advanced past the follicle, which remains undamaged by the cutting edges of the punch by passing through the slit. Once the punch has penetrated sufficiently, it can be partially rotated back and forth if desired, as schematically illustrated by the arrows, resulting in an arcuate cut in the tissue substantially circumscribing the curled follicle, while the follicle itself is spared by its clearance within the slit and isolation from the cutting edges. The intact hair follicle is then removed from the donor site for subsequent transfer to the recipient site.

To penetrate the skin, the punch could be manually pressed proximally by hand. Currently, it is believed that the use of an ultrasonic transducer to apply rapid, incremental, proximally-directed cutting force pulses to the tissue via the punch offers a more precisely controllable methodology for penetrating the tissue while the punch is positioned at the donor site and oriented so as to accommodate the follicle within the slit.

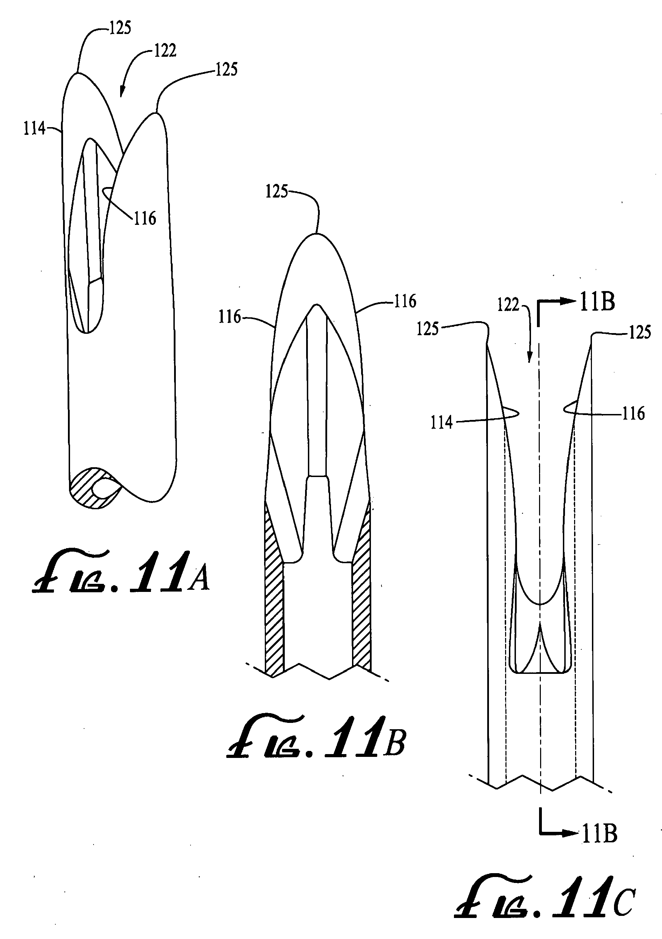

Turning to Figures 11A-C, another variation a follicle punch constructed in accordance with the invention, is illustrated. It should be noted that the interior surfaces of the punch are smooth; the apparent facets illustrated in Figures 11A and 11B are computer-generated "tangent" lines connoting a change in surface direction only.

The punch illustrated in Figures 11A-C comprises a pair of distally-extending circumferentially disposed, generally prong-like members 113 carrying distally diverging cutting edges 114, 116 and separated by a generally U-shaped follicle-accommodating slit 122. The cutting edge of each prong-like member is again preferably formed from the inside of the punch by grinding cutting edges outwardly from the interior region of the members. However, as noted earlier, the cutting edges can also be formed by laser cutting, water jet or abrasive jet cutting, chemical moulding, and/or other manufacturing process without departing from the scope of the present disclosure. The leading tips 125 of the punch
illustrated in Figures 11A-C are sharp rounded tips that make the initial penetration into the skin and tissue surrounding the targeted follicle.

To minimize the risk of the follicle being cut during the extraction process, the formation of the cutting edges may be limited to the first 0.060 inches (1.52 mm) or so from the distal tip 125 of the punch, so that the cutting edges pass the follicle during insertion of the punch at the donor site and any subsequent contact between the punch and follicle is not with a cutting edge. The cutting edge may however extend the entire length, or a different length, of the slits.

The gap between the prong-like members of the punch illustrated in Figures 11A-C is preferably 0.02-0.03 inches (0.51-0.76 mm) wide. It preferably extends proximally from the distal tip of the punch for about 0.12 to 0.16 inches (3.05 - 4.06 mm).

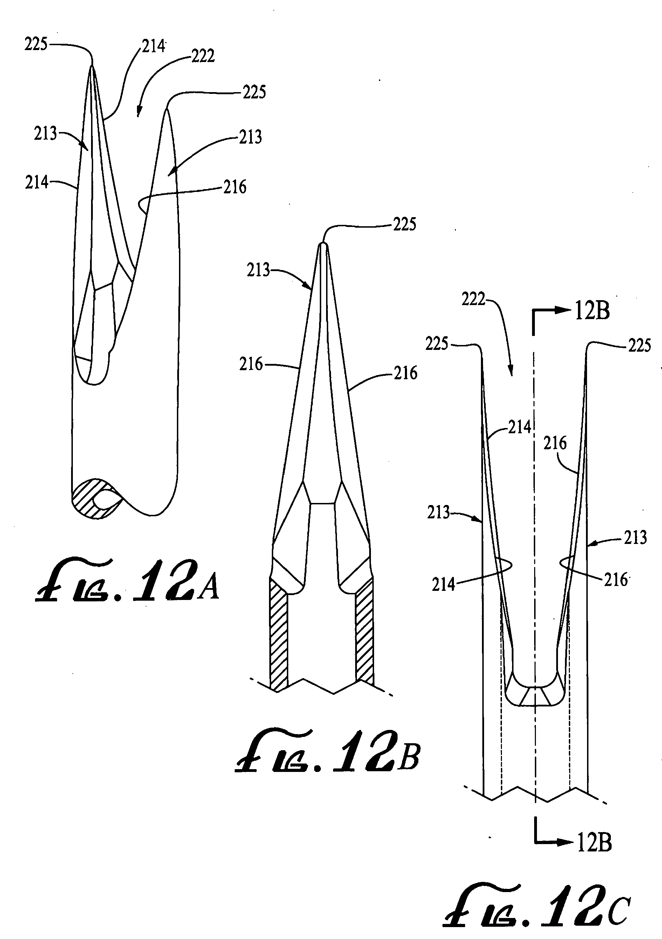

Turning to Figure 12A-C, another variation of the punch is illustrated, wherein the punch comprises a pair of distally-extending circumferentially disposed generally prong-like members 213 that carry distally-diverging cutting edges 214, 216 separated by a generally U-shaped follicle-accommodating slit. The leading tips 225 of the members 213 are sharp pointed tips. Each cutting edge 214, 216 is preferably formed from the inside of the punch by grinding cutting edges outwardly from the interior region of the prong-like members. However, as noted earlier, the cutting edges can also be formed by laser cutting, water jet or abrasive jet cutting, chemical moulding, and/or other manufacturing process without departing from the scope of the present disclosure. To minimize the risk of the follicle being cut during the extraction process, the formation of the cutting edges may be limited to the first 0.060 inches (1.52 mm) or so from the distal tip of the punch, so that the cutting edges pass the follicle during insertion of the punch at the donor site and any subsequent contact between the punch and follicle is not with a cutting edge. The cutting edge may however extend the entire length, or a different length, of the gap.

The gap between the prong-like members of the punch illustrated in Figures 12A-C is preferably 0.03 inches (0.076 mm) wide, and preferably extends proximally from the distal tip of the punch for about 0.16 inches (4.06 mm).

As with Figures 11A-B, it should be noted that the interior surfaces of the punch illustrated in Figures 12A-B are smooth, and that the apparent facets are computer-generated "tangent" lines connoting a change in surface direction only.

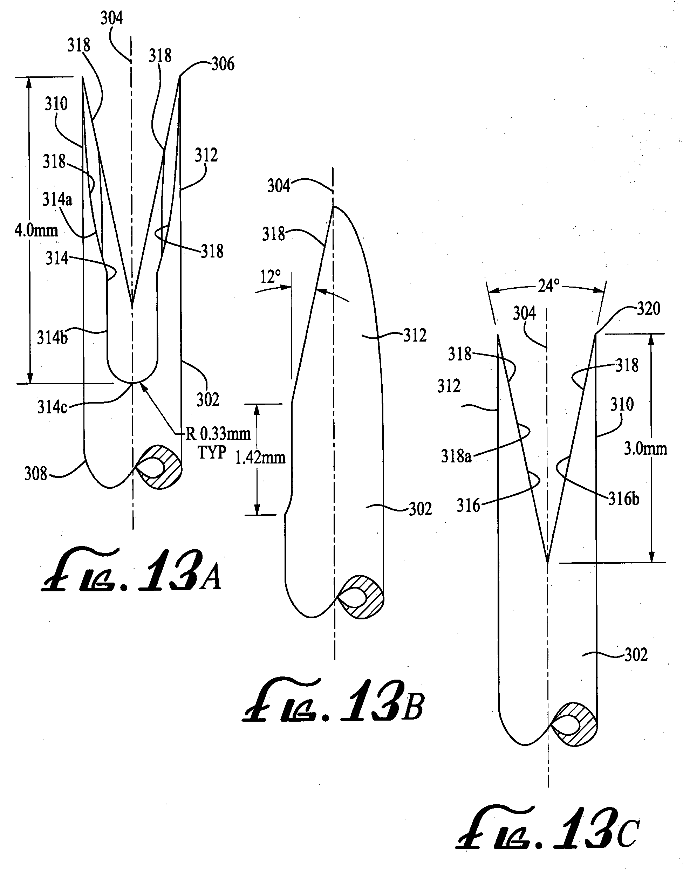

Figure 13A is a rear elevation view, in schematic, of the distal end region of the currently preferred punch for removing hair follicles from patients with straight or wavy hair, while Figure 13B is a left side elevation view, in schematic, of the distal end region illustrated in Figure 13A and Figure 13C is a front elevation view, in schematic, of the distal end region illustrated in Figure 13A. The distal end region illustrated in Figures 13A-C, comprises a generally tubular body 302 disposed about a generally longitudinal axis 304 between distal and proximal ends 306, 308, and having a distal cutting end region terminating distally in an opposing pair of distally-extending, generally prong-like members 310, 312 having posterior and anterior notches 314, 316 therebetween. The tubular body is preferably formed from stainless steel having a thickness in the range of 15-22 gauge (i.e., 0.0673 - 0.0299 inches; 1.709- 0.759 mm). For exemplary purposes, an 18 gauge thickness (0.0478 inches; 1.214 mm) will be used herein.

Each of the prong-like members carries cutting edges 318 preferably formed by a respective beveled surface within the generally tubular body that terminates at the cutting edge so that the cutting edge is formed at the outer surface (i.e., the outer diameter) of the generally tubular body. Preferably, the cutting edge extends completely around the prong-like member, and may be serrated or (preferably) non-serrated. The preferred bevel is created by sharpening the generally tubular body from the inside at a 15° angle.

As illustrated in Figures 13A and 13C, the currently preferred posterior notch 314 extends further in the proximal direction than the currently preferred anterior notch 316, and is shaped differently. Turning first to the preferred anterior notch best shown in Figure 13C, the notch 316 is generally V-shaped, comprising two proximally extending legs 316a, 316b that carry respective cutting edges formed by respective beveled surfaces. The apex of the "V" is approximately 0.118 inches (3 mm) proximally from the tip 320 of the prong-like member. The legs 316a, b of the notch each extend at a preferred angle of approximately 12° with respect to the longitudinal axis 304, resulting in angle of convergence of approximately 24°.

The currently preferred posterior notch 314, best illustrated in Figure 13A, comprises a generally "V"-shaped distal segment 314a, and a generally "U"-shaped proximal segment 314b that extends proximally from the distal segment. The U-shaped segment includes two proximally extending legs connected by a generally laterally extending base 314c at the proximal end of the notch. Preferably, and for reasons described later, the proximally-extending legs of the U-shaped segment carry respective sharpened cutting edges formed by respective beveled surfaces, while the base 314c lacks a sharpened cutting.

The currently preferred generally V-shaped distal segment 314a of the posterior notch is approximately 0.097 inches (2.46 mm) proximally of the tip 306. The base 314c lies approximately 0.157 inches (4 mm) proximally of the tip 306; i.e. 0.039 inches (1 mm) proximally further from the tip than the apex of anterior notch's apex. In practice, a difference in the range of 0-0.12 inches (0-3 mm) is acceptable for reasons described later. Preferably, the angle of convergence of the legs of the posterior notch's V-shaped segment is the same as that of the anterior notch's legs 316a,b.

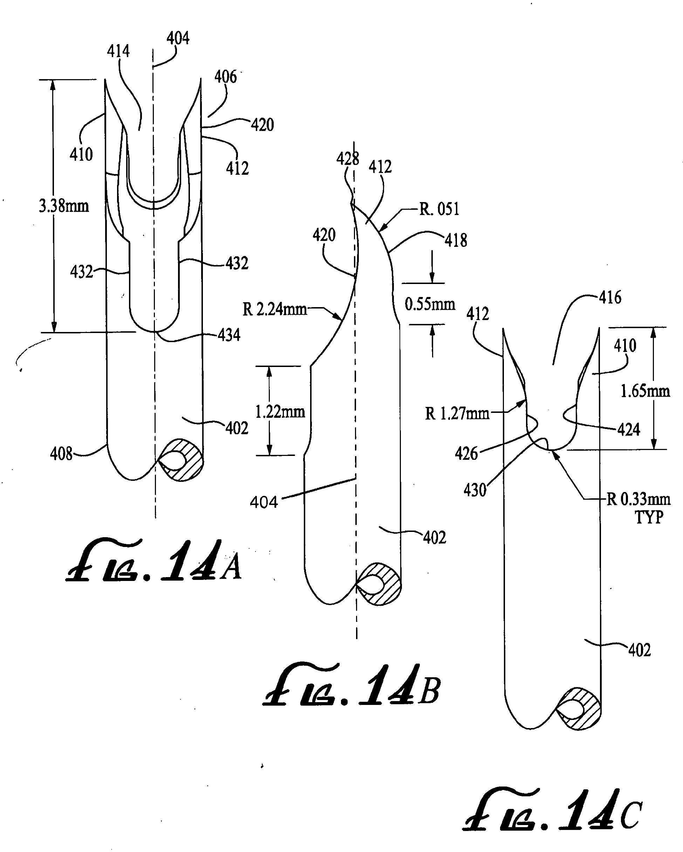

Figure 14A is a rear elevation view, in schematic, of the distal end region of the currently preferred punch constructed in accordance with the present disclosure for removing hair follicles from patients with kinky Afro-textured hair, while Figure 14B is a left side elevation view, in schematic, of the preferred distal end region illustrated in Figure 14A and Figure 14C is a front elevation view, in schematic, of the preferred distal end region illustrated in Figure 14A.

As illustrated in Figures 14A-C, the preferred punch's distal end region comprises a generally tubular body 402 disposed about a generally longitudinal axis 404 between distal and proximal ends 406, 408, and has a distal cutting end region terminating distally in an opposing pair of distally-extending, generally prong-like members 410, 412 having posterior and anterior notches 414, 416 therebetween. The prong-like members each have a convex curved anterior cutting edge 418 and a concave curved posterior cutting edge 420; the two cutting edges 418, 420 meeting at a cutting tip 428.

The prong-like members are preferably aligned with each other in the longitudinal and lateral directions (i.e., in the directions transverse to the longitudinal axis) as illustrated in Figure 14A-C, and have substantially the same shape as each other. As illustrated particularly in Figure 14B, the cutting tips 428 of the two prong-like members 410, 412 are thus aligned with one another, and in the particular embodiment illustrated are offset slightly from the longitudinal axis 404, towards the posterior side (i.e. the left-hand side as illustrated in Figure 14B) of the punch as viewed in side elevation along the plane of the longitudinal axis. However, in other embodiments it is proposed that the cutting tips 428 may be aligned with the longitudinal axis, when viewed from the side as in Figure 14B As shown most clearly in the side elevational view of Figure 14B, in the illustrated embodiment the concave posterior cutting edge 420 of each prong-like member 410, 412, extends from the distal end of the posterior notch 414 and through a transverse plane (i.e., orthogonal to the page) passing through the longitudinal axis 404 of the punch as viewed in side elevation, before curving back towards, and through, the same plane again.

By way of example, one preferred tubular body formed from 18-gauge stainless steel (a thickness of 0.0478 inches (1.214 mm)) terminates in prong-like members having a convex anterior cutting edge 418 characterized by a 0.051 inch (1.3 mm) radius of curvature and a concave posterior cutting edge 420 characterized by a 0.088 inch (2.24 mm) radius of curvature.

As with the punch of Figures 13A-C, the punch of Figures 14A-C preferably has a posterior notch 414 extending further proximately than the anterior notch 416 by a length in the range of 0 mm to 3 mm, inclusive. Turning first to the preferred anterior notch 416 best shown in Figure 14C, the notch 416 has a proximal segment that is generally U-shaped, comprising two proximally extending legs 424, 426 that preferably carry sharpened cutting edges. The legs 424, 426 are linked at their proximal end by a curved lateral segment 430 having, by way of example, a radius of curvature of 0.013 inches (0.33 mm) approximately 0.065 inches (1.65 mm) proximal of the cutting tip. The anterior notch 416 further comprises a distal generally "V"-shaped segment that interfaces with the proximal segment approximately 0.043 inches (1.09 mm) proximally from the tips 428.

The currently preferred posterior notch 414, best illustrated in Figure 14A, comprises a generally "U"-shaped posterior notch segment formed by two proximally extending legs 432 connected by a curved generally laterally extending base 434 at the proximal end of the notch 414. Preferably, and for reasons described later, the proximally-extending legs 432 of the U-shaped segment carry respective cutting edges formed by respective beveled surfaces, while the laterally-extending base 434 lacks a sharpened cutting edge.

The legs 432 of the illustrated posterior notch 414 interface with the prong-like members approximately 0.085 inches (2.16 mm) proximally from the tips 428. The laterally-extending base 434 is approximately 0.133 inches (3.38 mm) proximally from the tips. Accordingly, the posterior notch 414 extends approximately 0.083 inches (2.1 mm) further from the tips than the anterior notch 416.

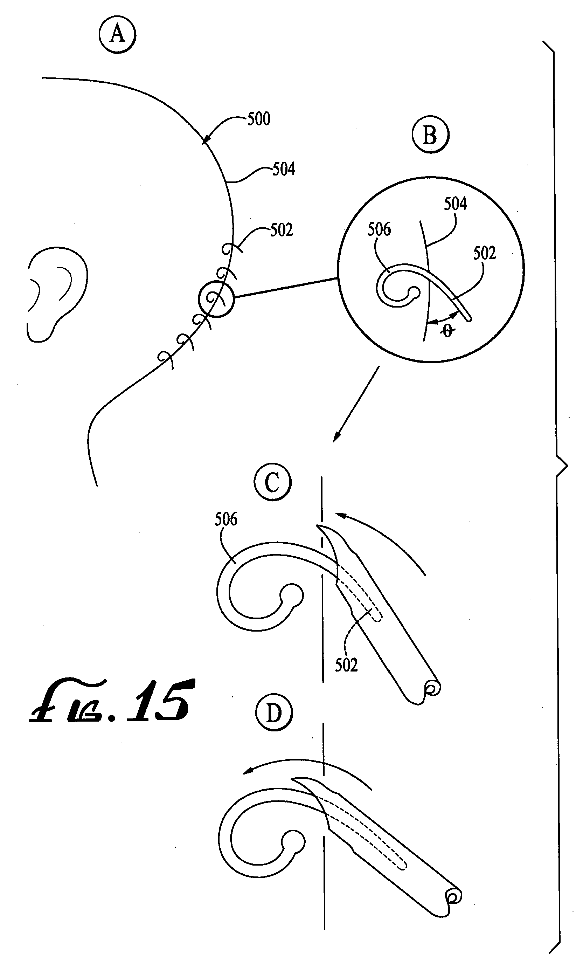

Figure 15 schematically illustrates (at "A") a subject's head 500 having a plurality of hairs 502 protruding from the skin 504. A hair 502 and its curled, subcutaneously-located follicle 506 is schematically illustrated in magnified form at "B". The hair follicle grow at an angle Θ to the skin, pointing from anterior to posterior.

As next schematically illustrated with greater magnification at "C", the preferred punch of Figures 14A-C is inserted into the skin at the donor site in such a way that the hair 502 enters the punch's interior. As the hair and follicle enter the punch's interior, its curl is accommodated by the posterior notch 414. Because the posterior notch 414 is sufficiently long in the proximal direction and its laterally-extending base 434 lacks a sharpened cutting edge, the curled hair 502 and follicle 506 is less likely to be damaged by contact with the edges of the posterior notch 414 during punch insertion. As the punch is further inserted from its positions in "C" and "D" to follow the follicle 506 as the prong-like members' cutting edges 418, 420 cut the surrounding tissue, the curled shape of the follicle 506 causes it to contact the posterior internal surface of the punch and, perhaps, the laterally-extending base 434 of the posterior notch 414. As the punch is advanced past the follicle 506, it remains undamaged by the cutting edges 418, 420 of the punch by passing through the posterior notch 414. Once the punch has penetrated sufficiently, it can be partially rotated back and forth if desired, resulting in an arcuate cut in the tissue substantially circumscribing the curled follicle, while the follicle itself is spared by its clearance within the posterior notch 414 and the lack of a cutting edge on the base member 434.

The placement of a "V"-shaped notch 416 on the anterior side of the punch illustrated in Figures 14A-C is preferred because the cutting edges circumscribing that notch shape cut more efficiently and controllably, and contact with the follicle (which points away from the anterior) is unlikely.

Those of ordinary skill in the art will recognize, however, that the anterior and posterior notches could be the same shape --- e.g., either generally V-shaped, generally "U" shaped, generally " 'V' plus 'U'-shaped, or other desired shape-and such combinations are within the scope of the present disclosure.

Regardless of the specific version of follicle punch utilized, the inclusion of an ultrasonic transducer coupled to the punch and selectively operable to enhance the cutting operation is desirable. The transducer is mounted within a handpiece to which the punch is attached in a manner analogous to the transducer, handpiece and scaler tip of an ultrasonic dental scaler.

The punch may be further mounted for reciprocating pivoting movement within the handpiece so as to move in such manner with or without ultrasonic vibratory movement. Likewise, the configuration may be such that ultrasonic vibratory movement can be generated with or without the pivoting movement.

In practice, it has been found that an adjustable degree of longitudinally reciprocating ultrasonic movement is desirable in that the appropriate degree of movement is a function of the subject's skin thickness and tissue, with higher settings being suitable when cutting through thicker skin or scar tissue for example. The use of the ultrasonic movement permits the surgeon or other operator of the equipment to better use his/her "fine motor" muscle movement to more precisely make the required incisions with greater sensitivity and finesse.

Although the present invention and its advantages have been described in detail, it should be understood that various changes, substitutions and alterations can be made herein without departing from the scope of the invention as will be defined by appended claims.

## Claims

1. A follicle punch comprising:
a generally tubular body (12) disposed about a generally longitudinal axis (11) between distal (16) and proximal (14) ends, and having a distal cutting end region terminating distally in an opposing pair of distally-extending, generally prong-like members (310, 312) having an anterior notch (316) and a posterior notch (314) therebetween, wherein
the prong-like members each have an interior cutting edge (314) and a posterior edge (318) meeting at a cutting tip (306), and **characterised in that**; the anterior and posterior edges are curved, and
one of said anterior and posterior notches extends proximately further than the other of said notches.

2. The punch of claim 1, wherein the anterior cutting edge of each prong-like member is convex, and the posterior cutting edge of each prong-like member is concave.

3. The punch of Claim 1 or claim 2, wherein the cutting tips are generally aligned with each other in the longitudinal direction.

4. The punch of any preceding claim, wherein the cutting tips are aligned with each other in a direction transverse to the longitudinal axis.

5. The punch of any preceding claim, wherein the prong-like members have substantially the same shape as each other.

6. The punch of any preceding claim, wherein the posterior notch extends proximately further than the anterior notch.

7. The punch of Claim 6 wherein the posterior notch extends further than the anterior 30 notch by a length in the range of 1 mm to 2 mm, inclusive.

8. The punch of any preceding claim, wherein posterior notch has a generally "V"-shaped distal segment, and a generally "U"-shaped proximal segment extending proximally from the distal segment.

9. The punch of Claim 8 wherein the U-shaped notch includes two proximally extending legs (424, 426) connected by a generally laterally extending base at the proximal end of the notch, and wherein at least a portion of the legs carry respective cutting edges formed by respective beveled surfaces.

10. The punch of Claim 8 or Claim 9, wherein the base (434) of the U-shaped notch lacks a cutting edge formed by a beveled cutting surface.

11. The punch of any preceding claim, wherein at least one of the notches is generally V-shaped.

12. The punch of Claim 11 wherein said V-shaped notch includes two proximally extending legs that carry respective cutting edges formed by respective beveled surfaces.

13. The punch of any preceding claim wherein the prong-like members include beveled surfaces terminating at the cutting edges.

14. The punch of Claim 13 wherein the beveled surfaces are formed on the inside surface of the tubular punch body.

15. A follicle punch comprising:
a generally tubular body (12) disposed about a generally longitudinal axis (11) between distal (16) and proximal (14) ends, and having a distal cutting end region terminating distally in an opposing pair of distally-extending, generally prong-like members (410, 412) having an anterior notch (416) and a posterior notch (414) therebetween, wherein
the prong-like members each have an anterior cutting edge (418) and a posterior edge (420) meeting at a cutting tip (428), and **characterised in that**; the anterior and posterior edges of the prongs are curved, and the anterior edges of the prongs are convex (418) and the posterior edges are concave (420), and
the convex curved anterior cutting edge has a smaller radius of curvature than the concave curved posterior edge.

## Patentansprüche

1. Follikelstanze, die umfasst:
einen allgemein rohrförmigen Körper (12), der zwischen einem distalen (16) und einem proximalen (14) Ende um eine allgemein in Längsrichtung verlaufende Achse (11) angeordnet ist und der ein distales Schneid-Endgebiet aufweist, das in einem gegenüberliegenden Paar distal verlaufender allgemein zinkenförmiger Elemente (310, 312) mit einer vorderen Kerbe (316) und mit einer hinteren Kerbe (314) dazwischen distal endet, wobei
die zinkenförmigen Elemente jeweils eine vordere Schneidkante (314) und eine hintere Kante (318), die sich bei einer Schneidspitze (306) treffen, aufweisen, und **dadurch gekennzeichnet, dass**
die vordere und die hintere Kante gekrümmt sind, und dass
die vordere oder die hintere Kerbe proximal weiter als die andere der Kerben verläuft.

2. Stanze nach Anspruch 1, wobei die vordere Schneidkante jedes zinkenförmigen Elements konvex ist und wobei die hintere Schneidkante jedes zinkenförmigen Elements konkav ist.

3. Stanze nach Anspruch 1 oder Anspruch 2, wobei die Schneidspitzen in der Längsrichtung allgemein aufeinander ausgerichtet sind.

4. Stanze nach einem vorhergehenden Anspruch, wobei die Schneidspitzen in einer Richtung quer zu der Längsachse aufeinander ausgerichtet sind.

5. Stanze nach einem vorhergehenden Anspruch, wobei die zinkenförmigen Elemente im Wesentlichen dieselbe Form aufweisen.

6. Stanze nach einem vorhergehenden Anspruch, wobei die hintere Kerbe proximal weiter als die vordere Kerbe verläuft.

7. Stanze nach Anspruch 6, wobei die hintere Kerbe um eine Länge in dem Bereich von einschließlich 1 mm bis 2 mm weiter als die vordere Kerbe verläuft.

8. Stanze nach einem vorhergehenden Anspruch, wobei die hintere Kerbe ein allgemein "V"-förmiges distales Segment und ein allgemein "U"-förmiges proximales Segment, das proximal von dem distalen Segment ausgeht, aufweist.

9. Stanze nach Anspruch 8, wobei die U-förmige Kerbe zwei proximal verlaufende Schenkel (424, 426) enthält, die durch eine allgemein quer verlaufende Basis bei dem proximalen Ende der Kerbe verbunden sind, und wobei wenigstens ein Abschnitt der Schenkel jeweilige Schneidkanten trägt, die durch jeweilige abgeschrägte Oberflächen gebildet sind.

10. Stanze nach Anspruch 8 oder Anspruch 9, wobei die Basis (434) der U-förmigen Kerbe keine durch eine abgeschrägte Schneidfläche gebildete Schneidkante aufweist.

11. Stanze nach einem vorhergehenden Anspruch, wobei wenigstens eine der Kerben allgemein V-förmig ist.

12. Stanze nach Anspruch 11, wobei die V-förmige Kerbe zwei proximal verlaufende Schenkel enthält, die jeweilige Schneidkanten tragen, die durch jeweilige abgeschrägte Oberflächen gebildet sind.

13. Stanze nach einem vorhergehenden Anspruch, wobei die zinkenförmigen Elemente abgeschrägte Oberflächen enthalten, die bei den Schneidkanten enden.

14. Stanze nach Anspruch 13, wobei die abgeschrägten Oberflächen auf der Innenoberfläche des rohrförmigen Stanzenkörpers gebildet sind.

15. Follikelstanze, die umfasst:
einen allgemein rohrförmigen Körper (12), der zwischen einem distalen (16) und einem proximalen (14) Ende um eine allgemein in Längsrichtung verlaufende Achse (11) angeordnet ist und der ein distales Schneid-Endgebiet aufweist, das in einem gegenüberliegenden Paar distal verlaufender allgemein zinkenförmiger Elemente (410, 412) mit einer vorderen Kerbe (416) und mit einer hinteren Kerbe (414) dazwischen distal endet, wobei
die zinkenförmigen Elemente jeweils eine vordere Schneidkante (418) und eine hintere Kante (420), die sich bei einer Schneidspitze (428) treffen, aufweisen, und **dadurch gekennzeichnet, dass**
die vordere und die hintere Kante der Zinken gekrümmt sind und dass die vorderen Kanten der Zinken konvex sind (418) und dass die hinteren Kanten konkav sind (420), und dass
die konvex gekrümmte vordere Schneidkante einen kleineren Krümmungsradius als die konkav gekrümmte hintere Kante aufweist.

## Revendications

1. Poinçon d'extraction folliculaire comportant :
une structure généralement tubulaire (12) disposée autour d'un axe généralement longitudinal (11) entre l'extrémité distale (16) et l'extrémité proximale (14), dont l'extrémité tranchante distale se termine en une paire de membres opposés généralement en forme de fourche et qui s'étendent vers l'extrémité distale (310, 312), et sont séparés par une encoche antérieure (316) et une encoche postérieure (314), où
les membres en forme de fourche arborent un bord tranchant antérieur (314) et un bord postérieur (318) qui se rejoignent au niveau d'une pointe coupante (306), et sont **caractérisés par** la forme incurvée des bords antérieur et postérieur, et
l'une desdites encoches antérieure et postérieure s'étend en direction de l'extrémité proximale plus loin que l'autre encoche.

2. Poinçon selon la Revendication 1, où le bord tranchant antérieur de chaque membre en forme de fourche est convexe, et le bord tranchant postérieur de chaque membre en forme de fourche est concave.

3. Poinçon selon la Revendication 1 ou 2, où les pointes coupantes sont généralement alignées l'une par rapport à l'autre dans le sens longitudinal.

4. Poinçon selon l'une des revendications précédentes, où les pointes coupantes sont alignées l'une par rapport à l'autre dans une direction transversale par rapport à l'axe longitudinal.

5. Poinçon selon l'une des revendications précédentes, où les membres en forme de fourche arborent quasiment la même forme.

6. Poinçon selon l'une des revendications précédentes, où l'encoche postérieure s'étend en direction de l'extrémité proximale plus loin que l'encoche antérieure.

7. Poinçon selon la Revendication 6, où l'encoche postérieure s'étend plus loin que l'encoche antérieure d'une distance comprise entre 1 et 2 mm.

8. Poinçon selon l'une des revendications précédentes, où l'encoche postérieure arbore un segment distal généralement en forme de « V », et un segment proximal généralement en forme de « U » qui s'étend vers l'extrémité proximale à partir du segment distal.

9. Poinçon selon la Revendication 8, où l'encoche en forme de U comporte deux broches qui s'étendent vers l'extrémité proximale (424, 426) et sont reliées par une base qui s'étend généralement sur l'axe latéral au niveau de l'extrémité proximale de l'encoche, et où au moins une partie des broches arborent des bords tranchants formés par leurs surfaces biseautées respectives.

10. Poinçon selon la Revendication 8 ou 9, où la base (434) de l'encoche en forme de U n'est pas pourvue d'un bord tranchant formé par une surface coupante biseautée.

11. Poinçon selon l'une des revendications précédentes, où au moins une des encoches est généralement en forme de V.

12. Poinçon selon la Revendication 11, où ladite encoche en forme de V comporte deux broches qui s'étendent vers l'extrémité proximale et arborent des bords tranchants formés par leurs surfaces biseautées respectives.

13. Poinçon selon l'une des revendications précédentes, où les membres en forme de fourche arborent des surfaces biseautées qui se terminent au niveau des bords tranchants.

14. Poinçon selon la Revendication 13, où les surfaces biseautées sont formées sur la surface interne du corps du poinçon tubulaire.

15. Poinçon d'extraction folliculaire comportant :
une structure généralement tubulaire (12) disposée autour d'un axe généralement longitudinal (11) entre l'extrémité distale (16) et l'extrémité proximale (14), dont l'extrémité tranchante distale se termine en une paire de membres opposés généralement en forme de fourche et qui s'étendent vers l'extrémité distale (410, 412), et sont séparés par une encoche antérieure (416) et une encoche postérieure (414), où
les membres en forme de fourche arborent un bord tranchant antérieur (418) et un bord postérieur (420) qui se rejoignent au niveau d'une pointe coupante (428), et sont **caractérisés par** la forme incurvée des bords antérieurs et postérieurs des fourches, la forme convexe des bords antérieurs des fourches (418) et la forme concave des bords postérieurs (420), et
le bord tranchant antérieur convexe affiche un rayon de courbure plus petit que celui du bord postérieur concave.
